(19) Europäisches Patentamt
European Patent Office
Office européen des brevets

(11) Publication number: 0 363 874 A1

# (12) EUROPEAN PATENT APPLICATION

(21) Application number: 89118729.6

(51) Int. Cl.[5]: C12N 11/10

(22) Date of filing: 09.10.89

Claims for the following Contracting States: ES + GR.

(30) Priority: 14.10.88 IT 2230888

(43) Date of publication of application: 18.04.90 Bulletin 90/16

(84) Designated Contracting States: AT BE CH DE ES FR GB GR IT LI LU NL SE

(71) Applicant: Etablissement TEXCONTOR
Egertstrasse 15
Vaduz(LI)

(72) Inventor: Conti, Franco
Via della Spiga, 33
I-20121 Milan(IT)

(74) Representative: Gervasi, Gemma et al
NOTARBARTOLO & GERVASI Srl Viale Bianca Maria 33
I-20122 Milan(IT)

## (54) Enzymes bound to a polymer matrix in water-soluble form.

(57) Water-soluble compounds consisting of proteolytic of enzymes stably grafted onto polyaldehyde matrices derived from starch. To prepare said compounds a polyaldehyde (DAS) is firstly prepared from starch by oxidation with an alkaline periodate to a content of -CHO of between $5x10^{-3}$ and $7x10^{-3}$ meq per mg of polymer, the enzyme is then grafted onto the DAS and the product obtained is finally reduced with an alkaline hydride.

EP 0 363 874 A1

# ENZYMES BOUND TO A POLYMER MATRIX IN WATER-SOLUBLE FORM

## Field of the invention

This invention relates to the preparation of enzymes bound to polymer matrices.

## Prior art

Numerous methods are known from the technical literature for preparing enzymes immobilized on polymer matrices of various kinds, and thus made insoluble.

It is known for example that papain, a protease extracted from Carica papaya and having a molecular weight of about 20,000, can be immobilized on a polyaldehyde matrix derived from starch or cellulose. The product obtained is insoluble in water and its activity is less than that of the free enzyme.

For various applications there is a currently felt requirement for enzymes stably bound to polymer matrices in water-soluble and active form.

## Summary of the invention

We have discovered water-soluble compounds consisting of proteolytic enzymes stably grafted onto a polymer matrix derived from starch, and a method for preparing said compounds. Said method is characterised by:

a) preparing a polyaldehyde (DAS) from starch by oxidation with an alkaline periodate to a content of -CHO of between $5x10^{-3}$ and $7x10^{-3}$ meg per mg of polymer;

b) grafting one or more enzymes onto the DAS with a weight ratio of enzyme to DAS of between 1% and 25%;

c) reducing with an alkaline hydride.

The product can be recovered by dialysis or lyophilization or similar methods of conventional use. In said product the enzyme is stably bound to DAS and maintains its activity for a long time.

## Detailed description of the invention

The characteristics and advantages of the method and products according to the present invention will be apparent from the following detailed description.

The method for preparing the compounds consisting of enzymes bound to the polymer matrix according to the invention is implemented in accordance with reaction scheme 1

SCHEME 1

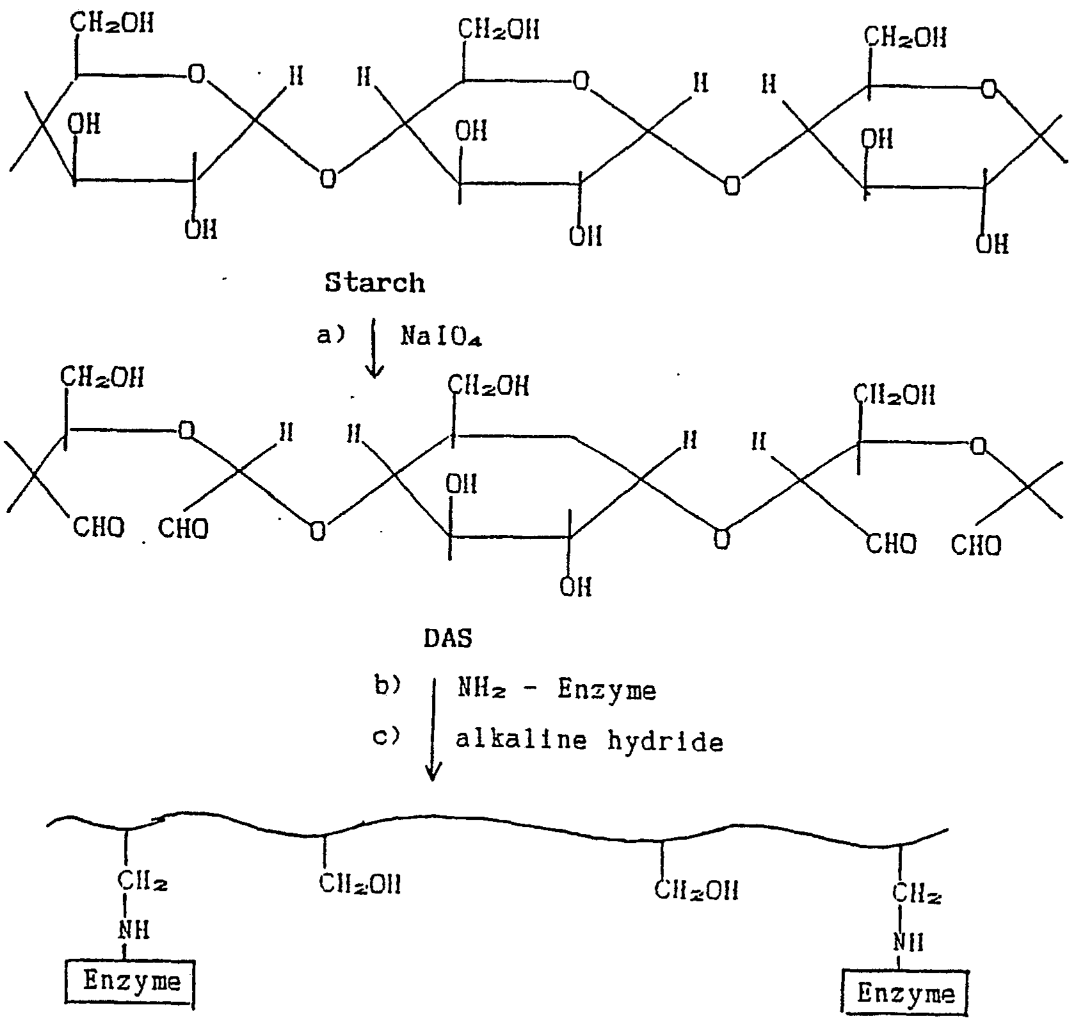


In the reaction of stage a) starch is oxidised with alkaline periodate to obtain the polyaldehyde (DAS). A starch is used having an average molecular weight of between 300,000 and 500,000 and the reaction is conducted in an aqueous environment under stirring, at ambient temperature and protected from light, using an aqueous mixture containing from 50 g/l to 100 g/l of starch and a ratio of alkaline periodate to starch of between 0,5/1 and 1/1 by weight. At the end of the reaction a small quantity of ethylene glycol is added to eliminate the unreacted oxidant excess.

The product can be separated by centrifuging and washed several times with hot water and then in succession with ethanol, acetone and ethyl ether centrifuging each time to remove the solvent.

Alternatively, teh product can be recovered by simply precipitating with an organic solvent miscible with the reaction medium.

In the reaction of stage b) the DAS is reacted with the enzyme so that the covalent bond $-CH=N-$ forms between the amino groups of the enzyme and an equivalent number of aldehyde groups in the DAS.

In this manner the enzyme is grafted onto the DAS.

This reaction is conducted in solution at pH 7 with a buffer and with an ionic force of between 0.01 and 0.1, containing between 40 and 60 g/l of DAS, under stirring at ambient temperature.

A weight ratio of enzyme to DAS of between 1% and 25% is used. Enzymes used are proteolytic enzymes and in particular papain and collagenase.

In stage c) the reaction mixture of stage b) is treated with alkaline hydride at ambient temperature, under stirring.

A weight ratio of hydride to DAS of between 10% and 15% is used.

Hydrides preferably used are sodium borohydride and sodium cyanoborohydride.

In this stage the $-CH=N-$ bond is reduced to $-CH_2NH-$ and in addition the aldehyde groups of the polymer matrix are reduced to alcohol groups which make the product water-soluble.

The product is recovered preferably by dialysis and lyophilization.

The essential condition for obtaining a water-soluble supported enzyme is that the DAS contains a minimum titre of aldehyde groups of not less than $5x10^{-3}$ meg of -CHO/mg of polymer. However, the titre of aldehyde groups in the DAS must not be too high, because even though an increase in this titre leads to a larger quantity of enzyme being bound, the enzyme activity is substantially reduced.

We have found that satisfactory water-solubility accompanied by good enzymatic activity is obtained in products deriving from DAS containing between $5x10^{-3}$ and $7x10^{-3}$ meg of -CHO/mg of polymer.

The enzyme is stably bound to DAS and no release of enzyme occurs for at least six months.

In addition the product obtained also has the advantage of a stability greater than that of the free enzyme both in aqueous solution and in a solid state.

The following examples of the preparation of the product according to the invention are given by way of illustration.

EXAMPLE 1

a) Oxidation of starch

73 g of sodium periodate (0.34 moles) are added to 100 g of starch (Noredux 150/T, average molecular weight 400,000) in 1.71 litres of deionized water under stirring at ambient temperature and protected from light. The mixture is left stirring protected from light at ambient temperature for two hours, after which 4.15 ml of ethylene glycol (74 mmoles) are added and the mixture left stirring at ambient temperature protected from light for 1 hour.

After this period the mixture, which still contains oxidants and therefore releases iodine when treated with $KI/H^+$, is in the form of an opalescent suspension which cannot be filtered through normal cellulose filters. The reaction product is centrifuged until sedimentation of the gelatinous mass, which is washed with hot water several times until the wash waters show no iodine reaction.

The product is washed successively with ethanol, acetone and ethyl ether, centrifuging each time to remove the solvent, and is finally recovered as a white solid (DAS) with a yield of 40% on the initial polymer and a concentration of $5.8x10^{-3}$ meg of CHO/mg of polymer.

The aldehyde concentration in the obtained product was determined by the following method:
250 mg of product are dispersed under stirring at ambient temperature in 5 ml of water and the pH is adjusted to 3.5 with 0.01 N HCl.
10 ml of 0.5 N hydroxylamine hydrochloride are then added to pH 3.5 (possible correction with HCl/0.01 N NaOH).

The pH is kept around 2 for six hours by adding 0.01 N NaOH (the quantity of NaOH added is taken into consideration in the final titration). At the end of the six hours, titration is carried out with 0.01 N NaOH to pH 3.5.

b) Grafting of papain onto the DAS

20 g of titrated DAS (113.2 meq CHO) are suspended under vigorous stirring in 400 ml of pH 7 phosphate buffer (ionic force 0.05).

The mixture is left stirring at ambient temperature until a homogeneous suspension of the product in solution is obtained (about 1 hour). 1 g of papain (Fluka-Puriss 3 U/mg; 0.0476 mmoles) is added and the mixture left stirring at ambient temperature for 20 minutes.

c) Reduction

2.4g of sodium borohydride are added in two portions with a 20 minute interval to the mixture of point b) and the mixture allowed to react for a further 80 minutes.

After this time, the reaction pH is adjusted to 7 with 0.1 N HCl and the mixture dialyzed and lyophilized. The reaction product is recovered with a yield of 60% (on the DAS) and is in the form of a white, soft solid with good water-solubility. The amount of grafted enzyme was found to be 50% after ultrafiltration through a suitable membrane.

The determination of the amount of grafted enzyme was done by the classical Lowry method (Lowry O.H., Rosebrough N.J., Farr A.L. and Randall L.J. (1951) J. Biol. Che, 193, 265-275).

Proteolytic activity of the product of Example 1

The proteolytic activity of the product of Example 1 was determined by the American Pharmacopeia method (USP XXI). Various DAS preparations of variable aldehyde titre were used, obtained by varying the reaction times, in order to establish a comparison between the aldehyde titre, the solubility of the enzymatic preparation and the activity of this latter.

The results are given in Table 1.

TABLE 1

| POLYMER TYPE | ALDEHYDE TITRE (meq CHO/mg polymer) | WATER SOLUBILITY | µg OF ACTIVE PAPAIN per 100 mg POLYMER |
|---|---|---|---|
| DAS.1 | $3.16 \times 10^{-3}$ | MICELLE | 800 |
| DAS.2 | $5.8 \times 10^{-3}$ | YES | 900 |
| DAS.3 | $8 \times 10^{-3}$ | YES | 200 |
| DAS.4 | $4.68 \times 10^{-3}$ | YES | 500 |

The product deriving from DAS.2, which has a content of -CHO in the range of the invention, is stable in solid state and maintains its activity for at least two years whereas free papain in the same conditions loses 35-45% of its activity.

The product deriving from DAS .2 is stable also in aqueous solution where no release of enzyme occurs for at least six months. The same product in aqueous solution maintains its activity for at least six months whereas free papain in the same conditions loses 40-50% of its activity.

EXAMPLE 2

Grafting of collagenase onto DAS and reduction

Following the procedure and reactant weight ratios used for grafting papain onto DAS (Example 1), 50 mg of collagenase (from Clostridium hystolyticum, molecular weight 105,000, Fluka cat. No. 27665, 0.3 U/mg) are grafted onto 1 g of DAS ($5.8 \times 10^{-3}$ meq CHO/mg) followed by reduction with sodium borohydride. The lyophilized product is recovered with a weight yield of 75% on the starting polymer and has good water-solubility.

The proteolytic activity of this product was determined by an experimental method already known in the literature (E. Wunsch, H.G. Heindrich (1963) Hoppe-Seyler's Z. Physiol. Chem. 333, 149-1511). In this manner it was found that 100 mg of supported enzyme possess the same proteolytic activity as 3.66 mg of free enzyme ( 0.011 U/mg of bound enzyme; 1 U corresponds to the quantity of enzyme which at pH 7.1 and 25°C hydrolyses 1, µmole of PZ-Pro-Leu-Gly-Pro-Arg per minute)

The product is stable in solid state and maintains its activity for at least two years whereas free collagenase in the same conditions loses 40-50% of its activity.

The product is stable also in aqueous solution at pH 7.4 where no release of enzyme occurs for at least six months. The same product in aqueous solution at pH 7.4 maintains its activity for at least six months whereas free collagenase in the same conditions loses 60-70% of its activity.

Physico-chemical characteristics of the products according to the invention

The lyophilized products of the invention have good watersolubility and are not precipitated by salting

($NH_4Cl$) or by the addition of ace tone or alcohols such as methanol and ethanol. In contrast, the free enzyme is precipitated by such treatment.

## Claims

1. A water-soluble compound consisting of a proteolytic enzyme stably grafted onto a polyaldehyde matrix derived from starch having an average molecular weight of between 300,000 and 500,000 and containing from $5x10^{-3}$ to $7x10^{-3}$ meq of -CHO per mg of polymer.

2. A water-soluble compound consisting of papain stably grafted onto a polyaldehyde matrix derived from starch having an average molecular weight of between 300,000 and 500,000 and containing from $5x10^{-3}$ to $7x10^{-3}$ meq of -CHO per mg of polymer.

3. A water-soluble compound consisting of collagenase stably grafted onto a polyaldehyde matrix derived from starch having an average molecular weight of between 300,000 and 500,000 and containing from $5x10^{-3}$ to $7x10^{-3}$ meq of -CHO per mg of polymer.

4. A method for preparing water-soluble compounds consisting of proteolytic enzymes s tably grafted onto a polymer matrix derived from starch, characterised by:

a) preparing a polyaldehyde (DAS) from starch by oxidation with an alkaline periodate to a content of -CHO of between $5x10^{-3}$ and $7x10^{-3}$ meq per mg of polymer;

b) grafting one or more enzymes onto the DAS with a weight ratio of enzyme to DAS of between 1% and 25%;

c) reducing with an alkaline borohydride.

5. A method as claimed in claim 4, characterised in that said starch used in stage a) has an average molecular weight of between 300,000 and 500,000

6. A method as claimed in claim 4, characterised in that said enzymes are papain and collagenase.

7. A method as claimed in claim 4, characterised in that the reaction of stage a) is conducted in an aqueous environment containing between 50 and 100 g/l of starch with a weight ratio of alkaline periodate to starch of between 0.5/1 and 1/1.

8. A method as claimed in claim 4, characterised in that the reaction of stage b) is conducted in a buffer solution (pH 7) with an ionic force of between 0.01 and 0.1 and containing between 40 and 60 g/l of DAS.

9. A method as claimed in claim 4, characterised in that the reaction of stage c) is conducted on the reaction mixture of stage b) with a weight ratio of alkalineborohydride to DAS of between 10% and 15%.

Claims for the following Contracting States: GR, ES.

1. A method for preparing water-soluble compounds consisting of proteolytic enzymes stably grafted onto a polymer matrix derived from starch, characterised by:

a) preparing a polyaldehyde (DAS) from starch by oxidation with an alkaline periodate to a content of -CHO of between $5x10^{-3}$ and $7x10^{-3}$ meq per mg of polymer;

b) grafting one or more enzymes onto the DAS with a weight ratio of enzyme to DAS of between 1% and 25%;

c) reducing with an alkaline borohydride.

2. A method as claimed in claim 1, characterised in that said starch used in stage a) has an average molecular weight of between 300,000 and 500,000

3. A method as claimed in claim 1, characterised in that said enzymes are papain and collagenase.

4. A method as claimed in claim 1, characterised in that the reaction of stage a) is conducted in an aqueous environment containing between 50 and 100 g/l of starch with a weight ratio of alkaline periodate to starch of between 0.5/1 and 1/1.

5. A method as claimed in claim 1, characterised in that the reaction of stage b) is conducted in a buffer solution (pH 7) with an ionic force of between 0.01 and 0.1 and containing between 40 and 60 g/l of DAS.

6. A method as claimed in claim 1, characterised in that the reaction of stage c) is conducted on the reaction mixture of stage b) with a weight ratio of alkaline borohydride to DAS of between 10% and 15%.

European Patent Office

# EUROPEAN SEARCH REPORT

Application Number

EP 89 11 8729

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | DE-A-2 919 622 (HENKEL KGaA) * Pages 5-9,12-17; claims * | 1,4,5,7-9 | C 12 N 11/10 |
| X | DIE MAKROMOLEKULARE CHEMIE, vol. 182, no. 6, 1981, pages 1641-1648; R.H. REINER: "HIO4-oxidized soluble polysaccharides as polyfunctional links for covalent binding of enzymes, 1. Preparation of polysaccharides and matrices for their binding" * Summary; introduction; table 1; page 1645 * | 1 | |
| A | CHEMICAL ABSTRACTS, vol. 80, no. 9, 4th March 1974, page 137, abstract no. 45218v, Columbus, Ohio, US; F.B. WEAKLEY et al.: "Binding of papain to dialdehyde starch", & BIOTECHNOL. BIOENG. 1973, 15(6), 1189-92 | | |
| | | | TECHNICAL FIELDS SEARCHED (Int. Cl.5) |
| | | | C 12 N |

The present search report has been drawn up for all claims

| Place of search | Date of completion of the search | Examiner |
|---|---|---|
| THE HAGUE | 09-11-1989 | ALVAREZ Y ALVAREZ C. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)